# EUROPÄISCHE PATENTSCHRIFT

(11) **EP 1 300 134 B1**
(45) Veröffentlichungstag und Bekanntmachung des Hinweises auf die Patenterteilung: **26.04.2006**
(21) Anmeldenummer: 02021429.2
(22) Anmeldetag: 25.09.2002
(51) Int. Cl.: A61Q 5/10, A61K 8/41, A61K 8/49, D06P 3/14

(54) **Mittel zum Färben von keratinhaltigen Fasern mit Isophthalaldehyden**
Agent for dyeing keratinous fibres with isophthalaldehydes
Agent pour teindre les fibres kératiniques avec des isophthalaldehydes

(30) Priorität: 04.10.2001 DE 10148843
(43) Veröffentlichungstag der Anmeldung: 09.04.2003
(73) Patentinhaber: Henkel Kommanditgesellschaft auf Aktien, 40589 Düsseldorf (DE)
(72) Erfinder: Gross, Wibke, Dr., 40549 Düsseldorf (DE); Oberkobusch, Doris, Dr., 40591 Düsseldorf (DE); Höffkes, Horst, Dr., 40595 Düsseldorf (DE)

(56) Entgegenhaltungen:
- DE-A- 19 859 809
- DE-U- 20 206 612
- US-A- 4 857 071
- US-A- 5 199 954
- DATABASE CA [Online] CHEMICAL ABSTRACTS SERVICE, COLUMBUS, OHIO, US; MATSUURA, MITSUNOBU ET AL: "Dyes with good storability and electrophotographic toners using the same" retrieved from STN Database accession no. 128:116285 XP002226549 & JP 09 328622 A (KONICA CO., JAPAN) 22. Dezember 1997 (1997-12-22)

## Beschreibung

Die Erfindung betrifft ein Mittel zum Färben von keratinhaltigen Fasern, insbesondere menschlichen Haaren, das eine Kombination aus mindestens einer Dicarbonylverbindung gemäß Formel I und CH-aciden Verbindungen und/oder Verbindungen mit primärer oder sekundärer Amino- oder einer Hydroxygruppe enthält, die Verwendung dieser Kombination als färbende Komponente in Haarfärbemitteln sowie ein Verfahren zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, -5-Amino-2-methylphenol, m-Amino-phenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Färbemittel, welche die unten näher beschriebene Kombination aus Dicarbonylverbindungen und Verbindungen mit primärer oder sekundärer Amino- oder einer Hydroxygruppe zum Färben von keratinhaltigen Fasern enthalten, sind bislang nicht bekannt.

In der Druckschrift US-A-5,199,954 werden Haarfärbemittel beschrieben, welche neben insbesondere 4-N,N-Dimethylaminobenzaldehyd oder 4-N,N-Dimethylaminozimtaldehyd ein p-Phenylendiaminderivat oder p-Aminophenol enthalten.

Die Druckschrift DE-A1-198 59 809 betrifft Haarfärbemittel, die aromatische Aldehyde oder Ketone jeweils mit einer quaternären Ammoniumgruppe, gegebenenfalls in Kombination mit CH-aktiven Verbindungen und/oder Verbindungen mit primärer oder sekundärer Amino- oder einer Hydroxygruppe, enthalten.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die Kombination aus Aldehyden der Formel I und CH-aciden Verbindungen und/oder Verbindungen mit primärer oder sekundärer Aminogruppe oder mit einer Hydroxygruppe sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agenzien soll dabei jedoch nicht prinzipiell ausgeschlossen werden.

Gegenstand der Erfindung ist ein Mittel zum Färben von keratinhaltigen Fasern, insbesondere menschlichen Haaren, enthaltend eine Kombination aus
A. mindestens einer Dicarbonylverbindung mit der Formel I (Komponente A), 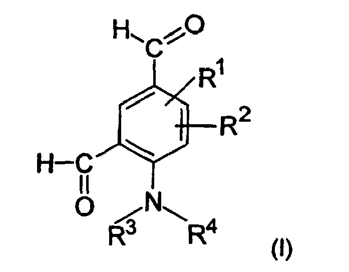 worin
   - R¹ und R² stehen unabhängig voneinander für ein Wasserstoffatom, ein Halogenatom, eine C₁-C₆-Alkylgruppe, eine C₂-C₆-Alkenylgruppe, eine C₁-C₆₋Alkoxygruppe, eine C₁-C₆-Hydroxyalkylgruppe, eine C₂-C₆-Polyhydroxyalkylgruppe, eine Hydroxy-C₁-C₆-alkoxygruppe, eine Hydroxygruppe, eine Sulfonsäuregruppe, eine Carboxygruppe, eine Nitrogruppe, eine Gruppe -NR⁵R₆, wobei R⁵ und R⁶ stehen unabhängig voneinander für eine C₁-C₆-Alkylgruppe, eine C₂-C₆₋Alkenylgruppe, eine C₁-C₆-Hydroxyalkylgruppe, eine C₁-C₆-Dialkylamino-C₁-C₆₋alkylgruppe oder eine Aryl-C₁-C₆-alkylgruppe oder wobei R⁵ und R⁶ zusammen mit dem Stickstoffatom einen 5-, 6- oder 7-gliedrigen Ring bilden könen,
      oder die Reste R¹ und R² zusammen einen 5-, 6- oder 7-gliedrigen, ankondensierten, aliphatischen oder aromatischen, carbozyklischen oder heterozyklischen Ring bilden,
   - R³ und R⁴ stehen unabhängig voneinander für eine C₁-C₆-Alkylgruppe, eine C₂-C₆₋Alkenylgruppe, eine C₁-C₆-Hydroxyalkylgruppe, eine C₁-C₆-Dialkylamino-C₁-C₆₋alkylgruppe oder eine Aryl-C₁-C₆-alkylgruppe oder zusammen mit dem Stickstoffatom einen 5-, 6- oder 7-gliedrigen Ring bilden,
   oder deren physiologisch verträglichen Salzen und
B. mindestens einer Verbindung (Komponente B) ausgewählt aus
   (a) CH-aciden Verbindungen,
   (b) Verbindungen mit primärer oder sekundärer Amino- oder einer Hydroxygruppe, ausgewählt aus aromatischen Hydroxyverbindungen, primären oder sekundären aromatischen Aminen und stickstoffhaltigen heterozyklischen Verbindungen,
   und/oder deren physiologisch verträglichen Salzen.

Beispiele für die als Substituenten im Rahmen dieser Anmeldung genannten C₁-C₆₋Alkylreste sind die Gruppen Methyl, Ethyl, Propyl, Isopropyl, n-Butyl, tert-Butyl, n-Pentyl, neo-Pentyl und n-Hexyl. Ethyl und Methyl sind bevorzugte Alkylreste. Beispiele für bevorzugte C₂-C₆-Alkenylreste sind Vinyl, Allyl und Butenyl. Erfindungsgemäß bevorzugte C₁-C₆-Alkoxyreste sind beispielsweise eine Methoxy- oder eine Ethoxygruppe. Weiterhin können als bevorzugte Beispiele für eine C₁-C₆₋Hydroxyalkylgruppe eine Hydroxymethyl-, eine 2-Hydroxyethyl-, eine 2-Hydroxypropyl, eine 3-Hydroxypropyl- oder eine 4-Hydroxybutylgruppe genannt werden. Eine 2-Hydroxyethylgruppe ist besonders bevorzugt. Beispiele für eine bevorzugte C₂-C₆₋Polyhydroxyalkylgruppe sind die 1,2-Dihydroxyethylgruppe und die 2,3-Dihydroxypropylgruppe. 2-Methoxyethyl, 2-Ethoxyethyl, 3-Ethoxypropyl und 3-Methoxypropyl sind Beispiele für eine C₁-C₆-Alkoxy-C₁-C₆-alkylgruppe. Bevorzugte Hydroxy-C₁-C₆-Alkoxygruppen sind 2-Hydroxyethoxy, 2-Hydroxypropyl und 3-Hydroxypropyl. Bevorzugte Aryl-C₁-C₆-alkylgruppen sind Benzyl, 2-Phenylethyl, 5-Phenylpropyl, wobei Benzyl besonders bevorzugt ist. Beispiele für bevorzugte C₁-C₆₋Dialkylamino-C₁-C₆-alkylgruppen sind 2-(N,N-Dimethylamino)ethyl, 2-(N,N-Diethylamino)ethyl, 3-(N,N-Dimethylamino)propyl und 3-(N,N-Diethylamino)propyl. Beispiele für Halogenatome sind F-, Cl-, oder Br-Atome, wobei Cl-Atome ganz besonders bevorzugt sind. Die weiteren verwendeten Begriffe leiten sich erfindungsgemäß von den hier gegebenen Definitionen ab.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasem, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern verwendet werden.

Bevorzugt eingesetzte Verbindungen mit der Formel I sind 4-Dimethylamino-6-methyl-isophthalaldehyd, 4-Dimethylamino-6-methoxy-isophthalaldehyd und 4-Dimethylamino-isophthalaldehyd.

Die Substanzen gemäß Formel I sind zum großen Teil literaturbekannt oder im Handel erhältlich oder nach bekannten Syntheseverfahren herstellbar.

Als CH-acide werden im Allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Erfindungsgemäß sind die CH-aciden Verbindungen bevorzugt ausgewählt aus Verbindungen gemäß einer der Formeln C1 bis C21:

M¹ - CH₂ - M² (C1)

worin
- M¹ eine Gruppe -COM³, COOM³, S(O)M³ oder SO₂M³ ist, worin M³ für ein Wasserstoffatom oder eine C₁-C₆-Alkylgruppe steht, oder eine Gruppe - C(M⁴)=C(C≡N)₂ bedeutet, worin M⁴ für ein Wasserstoffatom, eine C₁-C₄-Alkylgruppe oder eine Arylgruppe steht,
- M² die gleiche Bedeutung wie M¹ hat oder eine Cyanogruppe, eine substituierte oder unsubstituierte Arylgruppe oder Aryl-C₁-C₄-alkylgruppe, ein substituierter oder unsubstituierter, gesättigter oder ungesättigter Heterozyklus ist,
Verbindungen mit der Formel C2 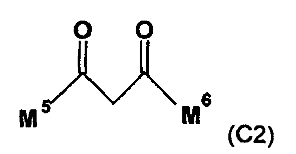 worin
- M⁵ eine Cyanogruppe, eine substituierte oder unsubstituierte Arylgruppe oder eine Aryl-C₁-C₄-alkylgruppe, ein substituierter oder unsubstituierter, gesättigter oder ungesättigter Heterozyklus oder eine Gruppe -COM⁷ oder COOM⁷ ist, worin M⁷ für ein Wasserstoffatom oder eine C₁-C₆-Alkylgruppe steht,
- M⁶ eine substituierte oder unsubstituierte C₁-C₆-Alkylgruppe, eine Acetyloxygruppe, eine C₃-C₆-Cycloalkylgruppe, eine substituierte oder unsubstituierte Arylgruppe oder Aryl-C₁-C₄-alkylgruppe, eine substituierte oder unsubstituierte Aminoarylgruppe, ein substituierter oder unsubstituierter, gesättigter oder ungesättigter Heterozyklus ist,
Verbindungen mit der Formel C3 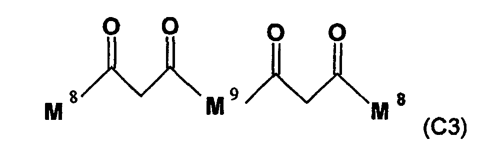 worin
- M⁸ eine Cyanogruppe, eine substituierte oder unsubstituierte Arylgruppe oder Aryl-C₁-C₄-alkylgruppe, ein substituierter oder unsubstituierter, gesättigter oder ungesättigter Heterozyklus oder eine Gruppe -COM¹⁰ oder COOM¹⁰ ist, worin M¹⁰ für ein Wasserstoffatom oder eine C₁-C₆-Alkylgruppe steht,
- M⁹ eine substituierte oder unsubstituierte Arylgruppe oder Aryl-C₁-C₄-alkylgruppe, eine substituierte oder unsubstituierte Aminoarylgruppe, ein substituierter oder unsubstituierter, gesättigter oder ungesättigter Heterozyklus ist,
Pyrazolderivate (a), die ausgewählt sein können aus den folgenden Formeln C4 und C5 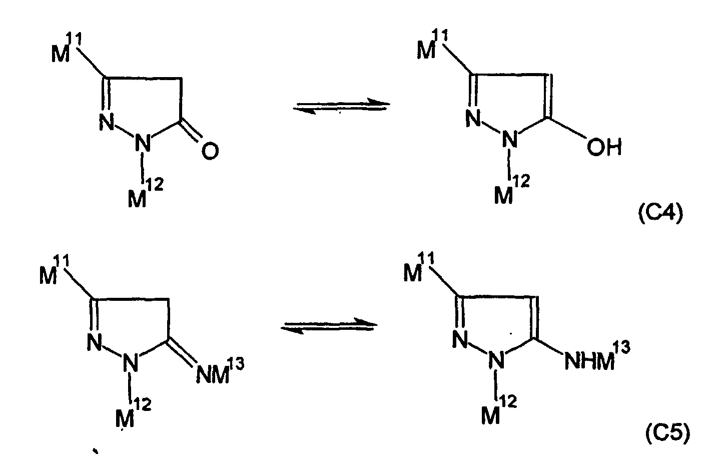 worin
- M¹¹ und M¹² unabhängig voneinander für eine substituierte oder unsubstituierte C₁₋C₆-Alkylgruppe, eine Acetyloxygruppe, eine C₃-C₆-Cycloalkylgruppe, eine substituierte oder unsubstituierte Arylgruppe oder Aryl-C₁-C₄-alkylgruppe, eine substituierte oder unsubstituierte Aminoarylgruppe, einen substituierter oder unsubstituierter, gesättigten oder ungesättigten Heterozyklus stehen,
- M¹³ ein Wasserstoffatom oder eine substituierte oder unsubstituierte C₁-C₆₋Alkylgruppe ist,
(b) zwei über M¹¹ oder M¹² gebundene Pyrazolringe mit der Formel C4 oder C5
Barbitursäurederivate mit der folgenden Formel C6 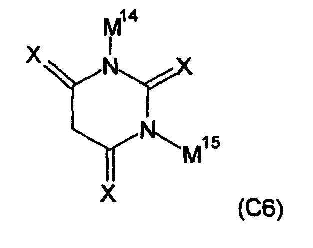 worin
- M¹⁴ und M¹⁵ stehen unabhängig voneinander für eine C₁-C₆-Alkylgruppe, eine C₂-C₆₋Alkenylgruppe, eine C₃-C₆-Cycloalkylgruppe, eine Aryl-C₁-C₄-alkylgruppe, eine substituierte oder unsubstituierte Arylgruppe, oder einen über die Reste M¹⁴ oder M¹⁵ gebundenen Bicyclus,
- X steht für ein Sauerstoff- oder ein Schwefelatom,
Pyridinderivate mit der Formeln C7a und C7b 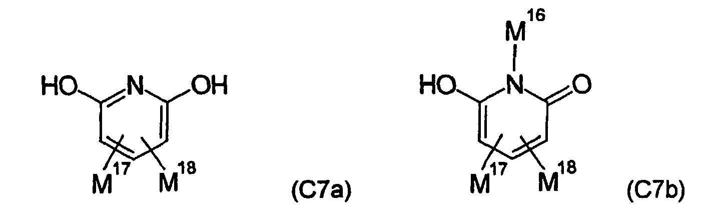 worin
- M¹⁶ eine substituierte oder unsubstituierte C₁-C₆-Alkylgruppe oder substituierte oder unsubstituierte Arylgruppe ist,
- M¹⁷ ein Wasserstoffatom, eine substituierte oder unsubstituierte C₁-C₆-Alkylgruppe oder eine substituierte oder unsubstituierte Arylgruppe ist,
- M¹⁸ ein Wasserstoffatom, eine Cyanogruppe, eine substituierte oder unsubstituierte C₁-C₆-Alkylgruppe, eine Gruppe COOM¹⁹, worin M¹⁹ ein Wasserstoffatom oder eine substituierte oder unsubstituierte C₁-C₆-Alkylgruppe bedeutet, ist,
Verbindungen mit der folgenden Formel C8 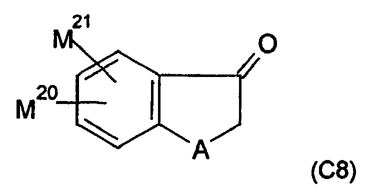 worin
- A steht für ein Sauerstoffatom, ein Schwefelatom, eine Sulfoxylgruppe, eine Sulfonylgruppe oder eine Gruppe NM^{20a}, worin M^{20a} ein Wasserstoffatom, eine substituierte oder unsubstituierte C₁-C₆-Alkylengruppe bedeutet,
- M²⁰ und M²¹ stehen unabhängig voneinander für ein Wasserstoff-, ein Chlor-, ein Bromatom, eine Hydroxygruppe, eine Nitrogruppe, eine C₁-C₈-Alkylgruppe, eine C₁₋C₆-Alkoxy-, eine Carboxamid-, eine Sulfonamid-, eine Carboxyl-, eine C₁-C₄-Acyl, eine Cyanogruppe oder eine Aminogruppe -NM²²M²³, in der M²² und M²³ unabhängig voneinander stehen für ein Wasserstoffatom oder eine C₁-C₆-Alkylgruppe,
Verbindungen mit den Formeln C9 und C10 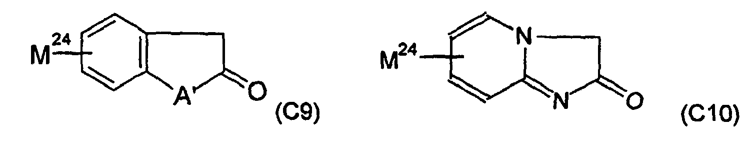 worin
- A' steht für ein Sauerstoffatom, ein Schwefelatom oder eine Gruppe NM²⁵, worin M²⁵ ein Wasserstoffatom oder eine substituierte oder unsubstituierte C₁-C₆-Alkylgruppe bedeutet,
- M²⁴ steht für ein Wasserstoff-, ein Chlor-, ein Bromatom, eine Hydroxygruppe, eine Nitrogruppe, eine C₁-C₆-Alkyl-, eine C₁-C₆-Alkoxy-, eine Carboxamid-, eine Sulfonamid-, eine Carboxyl-, eine C₁-C₄-Acyl, eine Cyanogruppe oder eine Aminogruppe -NM²⁸M²⁷, in der M²⁶ und M²⁷ unabhängig voneinander stehen für Wasserstoff und eine C₁-C₆-Alkylgruppe
Verbindungen mit der Formel C11 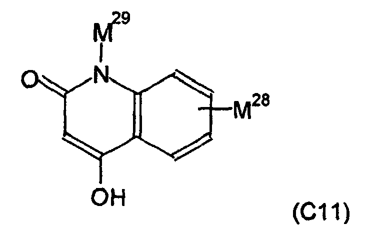 worin
- M²⁸ ein Wasserstoffatom, eine Hydroxygruppe, eine substituierte oder unsubstituierte C₁-C₆-Alkylgruppe oder eine substituierte oder unsubstituierte Aryl- oder C₁-C₆₋Alkylarylgruppe ist;
- M²⁹ steht für ein Wasserstoffatom oder eine C₁-C₄-Alkylgruppe
Indandionderivate mit der Formel C12 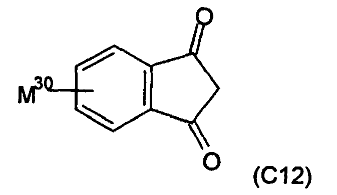 worin
- M³⁰ ein Wasserstoff-, ein Chlor-, ein Bromatom, eine Nitro-, eine C₁-C₆-Alkyl-, eine C₁-C₆-Alkoxy-, eine Carboxamid-, eine Sulfonamid- oder eine Cyanogruppe ist,
Verbindungen mit der Formel C13 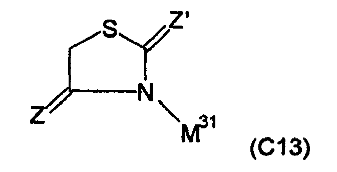 worin
- Z steht für ein Sauerstoffatom oder eine Gruppe NM³², worin M³² ein Wasserstoffatom oder eine C₁-C₈-Alkylgruppe bedeutet,
- Z' steht für ein Schwefelatom oder eine Gruppe NM³³, worin M³³ ein Wasserstoffatom oder eine C₁-C₆-Alkylgruppe bedeutet,
- M³¹ steht für ein Wasserstoffatom, eine C₁-C₆-Alkylgruppe oder eine C₁-C₄-Carboxy-alkylgruppe,
Dioxopyrazolverbindungen mit der Formel C14 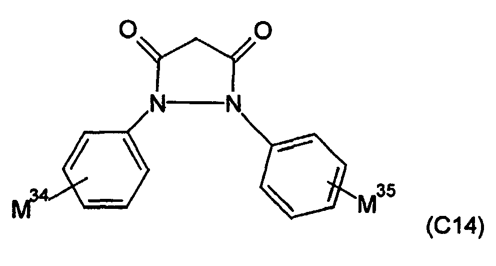 worin
- M³⁴ und M³⁵ stehen unabhängig voneinander für ein Wasserstoff-, ein Chlor-, ein Bromatom, eine Hydroxygruppe, eine Nitrogruppe, eine C₁-C₆-Alkyl-, eine C₁-C₆₋Alkoxy-, eine Carboxamid-, eine Sulfonamid-, eine Carboxyl-, eine C₁-C₄-Acyl, eine Cyanogruppe oder eine Aminogruppe -NM³⁶M³⁷, in der M³⁶ und M³⁷ unabhängig voneinander stehen für Wasserstoff oder eine C₁-C₆-Alkylgruppe,
5-Oxoimidazolderivate mit der Formel C15 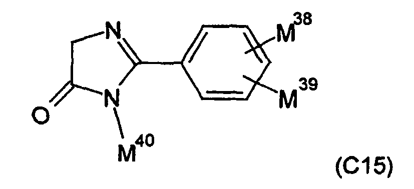 worin
- M³⁸ und M³⁹ stehen unabhängig voneinander für ein Wasserstoff-, ein Chlor-, ein Bromatom, eine Hydroxygruppe, eine Nitrogruppe, eine C₁-C₆-Alkyl-, eine C₁-C₆₋Alkoxy-, eine Carboxamid-, eine Sulfonamid-, eine Carboxyl-, eine C₁-C₄-Acyl, eine Cyanogruppe oder eine Aminogruppe -NM⁴¹M⁴², in der M⁴¹ und M⁴² unabhängig voneinander stehen für ein Wasserstoffatom oder eine C₁-C₆-Alkylgruppe,
- M⁴⁰ steht für ein Waserstoffatom oder eine C₁-C₆-Alkylgruppe,
Derivate von Dehydrobutyrolacton mit der Formel C16 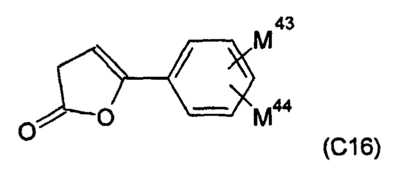 worin
- M⁴³ und M⁴⁴ unabhängig voneinander stehen für ein Wasserstoff-, ein Chlor-, ein Bromatom, eine Hydroxygruppe, eine Nitrogruppe, eine C₁-C₆-Alkyl-, eine C₁-C₆₋Alkoxy-, eine Carboxamid-, eine Sulfonamid-, eine Carboxyl-, eine C₁-C₄-Acyl, eine Cyanogruppe oder eine Aminogruppe -NM⁴⁵M⁴⁶, in der M⁴⁵ und M⁴⁶ unabhängig voneinander stehen für ein Wasserstoffatom oder eine C₁-C₆-Alkylgruppe
Verbindungen mit der Formel C17 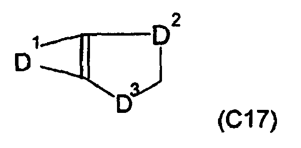 worin
- D¹ ist ein ankondensierter aromatischer oder heteroaromatischer Ring,
- D² ist eine Carbonylgruppe, eine Gruppe C=CD'D'' oder eine Gruppe CD'D", in welchen D' oder D" jeweils ein Substituent mit einer Hammett-Konstante zwischen 0,4 und 2,0 oder beide Substituenten in der Summe eine Hammett-Konstante zwischen 0,4 und 2,0 aufweisen;
- D³ steht für eine Carbonylgruppe, ein Sauerstoff-, ein Schwefelatom, eine Gruppe NM⁴⁷, wenn D² nicht Sauerstoff ist, oder eine Gruppe C=S, eine Gruppe C=NR⁴⁸, eine Sulfinylgruppe, eine Sulfonylgruppe, wobei R⁴⁷ und R⁴⁸ unabhängig voneinander ein Wasserstoffatom oder einen C₁-C₄-Alkylrest bedeuten,
Hydroxypyrimidinderivate mit der Formel C18 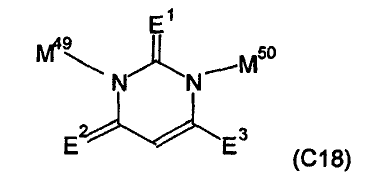 worin
- M⁴⁹ und M⁵⁰ unabhängig voneinander ein Wasserstoffatom oder eine substituierte oder unsubstituierte C₁-C₆-Alkylgruppe sind,
- E¹ für ein Sauerstoff, ein Schwefelatom oder eine Gruppe NH steht,
- E² für eine Gruppe NH oder ein Sauerstoffatom steht,
- E³ für eine Aminogruppe oder eine Hydroxygruppe steht,
mit der Maßgabe, daß
a) wenn E¹ und E² für ein Sauerstoffatom stehen, E³ keine Hydroxygruppe ist, und
b) wenn E¹ ein Schwefelatom und E² ein Sauerstoffatom ist, E³ keine Hydroxygruppe ist,
quaternierte Stickstoffverbindungen der Formel C19 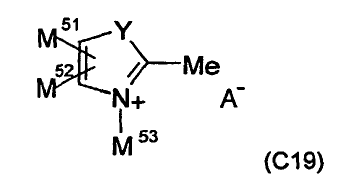 worin,
- M⁵¹ und M⁵² stehen unabhängig voneinander für ein Wasserstoffatom, ein Halogenatom, eine Hydroxygruppe, eine C₁-C₄-Hydroxyalkylgruppe, eine C₁-C₆₋Aminoalkylgruppe, eine C₁-C₄-Dialkylamino-C₁-C₄-alkylgruppe, eine lineare oder verzweigte C₁-C₆-Alkylgruppe, eine C₂-C₆-Alkenylgruppe, eine gegebenenfalls substituierte Arylgruppe, eine Sulfonsäuregruppe, eine Carboxylgruppe, eine Formylgruppe, eine Nitrogruppe, eine Cyanogruppe oder eine Gruppe -NM⁵⁴M⁵⁵, wobei M⁵⁴ und M⁵⁵ stehen unabhängig voneinander für ein Wasserstoffatom, eine C₁-C₆-Alkylgruppe, eine C₂-C₆-Alkenylgruppe, eine Aryl-C₁-C₄-alkylgruppe oder eine C₁-C₄-Hydroxyalkylgruppe, wobei M⁵¹ und M⁵² zusammen einen ankondensierten 5- oder 6-gliedrigen, aliphatischen oder aromatischen bzw. heteroaromatischen Ring bilden können, welcher wiederum mit den Resten M⁵⁶ und M⁵⁷ substituiert ist, wobei M⁵⁶ und M⁵⁷ stehen unabhängig voneinander für die Reste, die unter M⁵¹ definiert sind,
- M⁵³ steht für ein Wasserstoffatom, eine C₁-C₄-Hydroxyalkylgruppe, eine C₁-C₆₋Aminoalkylgruppe, eine C₁-C₄-Dialkylamino-C₁-C₄-alkylgruppe, eine lineare oder verzweigte C₁-C₆-Alkylgruppe, eine C₂-C₆-Alkenylgruppe, eine gegebenenfalls substituierte Arylgruppe, eine C₁-C₄-Sulfonylalkylgruppe, eine C₁-C₄₋Carboxyalkylgruppe oder eine C₂-C₆-Polyhydroxyalkylgruppe,
- Y steht für ein Sauerstoffatom, ein Schwefelatom, eine gegebenenfalls substituierte Methylengruppe oder eine Gruppe NM⁶⁰, wobei M⁶⁰ für die gleichen Gruppen stehen kann, die unter M⁵⁵ definiert sind,
- A steht für ein Chlorid, Bromid, lodid, Hexafluorophosphat, Tetrachlorozinkat, Tetrafluoroborat, Trifluormethylsulfonat, Methylsulfonat oder p-Toluolsulfonat,
Oniumverbindungen der Formeln C20 und C21 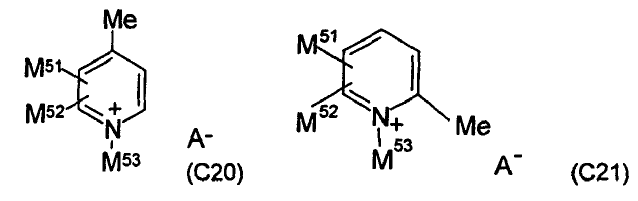 wobei M⁵¹, M⁵², M⁵³ und A⁻ aus den Gruppen ausgewählt werden, die unter Verbindung C19 definiert sind.

Unter CH-acide Verbindungen fallen erfindungsgemäß auch Enamine, die aus quaternierten N-Heterocyclen mit einer in Konjugation zum quartären Stickstoff stehenden CH-aciden Alkylgruppe durch alkalische Behandlung entstehen. Als Beispiele für geeignete Enamine können Verbindungen mit der allgemeinen Formel C22 genannt werden, 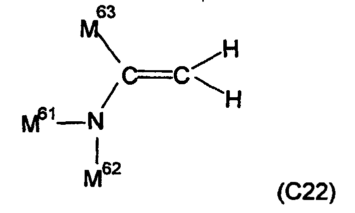 worin
- M⁶¹ steht für einen aromatischen Rest, insbesondere für einen gegebenenfalls mit einer C₁-C₄-Alkyl-, C₁-C₄-Hydroxyalkyl-, Hydroxy-, Methoxy- oder Halogengruppe substituierten 5-gliedrigen oder 6-gliedrigen Arylrest, vorzugsweise ein Phenylrest, oder einen 5-gliedrigen oder 6-gliedrigen, ankondensierten, aliphatischen oder aromatischen, carbozyklischen oder heterozyklischen Ring, vorzugsweise einen Phenylrest, einen Chinolin- oder Pyridylrest,
- M⁶² steht für ein Wasserstoffatom, eine lineare oder verzweigte C₁-C₈ Alkyl-, eine lineare oder verzweigte C₁-C₈-Hydroxyalkyl- oder eine C₁-C₈-Alkoxyalkylgruppe, wobei zwischen den C-Atomen der Alkylkette ein Sauerstoffatom sitzen kann, und
- M⁶³ steht für eine lineare oder verzweigte C₁-C₈-Alkylgruppe, eine C₁-C₆-Alkoxy-C₁₋C₆-alkylgruppe, eine C₁-C₆-Alkylamino-C₁-C₆-alkylgruppe, eine C₁-C₆-Alkylmercapto-C₁-C₆-alkylgruppe, eine C₁-C₆-Alkoxy-C₁-C₆-alkylengruppe, eine C₁-C₆-Alkylamino-C₁-C₆-alkylengruppe, eine C₁-C₆-Alkylmercapto-C₁-C₆-alkylengruppe, eine geradkettige oder verzweigte C₁-C₈-Alkylengruppe, oder ein Sauerstoffatom, ein Stickstoffatom oder ein Schwefelatom ist, mit der Maßgabe, daß die Reste M⁶¹ und M⁶³ gemeinsam mit dem Stickstoffatom und dem Kohlenstoffatom der Enamingrundstruktur eine cyclische Verbindung bilden, wenn M⁶³ gleich einer linearen oder verzweigten C₁-C₈-Alkylengruppe, einer C₁-C₈-Alkoxyalkylengruppe, eine C₁-C₆-Alkylamino-C₁-C₆-alkylengruppe, eine C₁-C₆-Alkylmercapto-C₁-C₆₋alkylengruppe, einem Sauerstoffatom, einem Stickstoffatom oder einem Schwefelatom ist, wobei vorzugsweise M⁶³ am aromatischen Rest M⁶¹ mit dem Kohlenstoff verbunden ist, der in ortho-Stellung zum Enamin-substituierten Kohlenstoff steht.

In einer besonders bevorzugten Ausführungsform sind die CH-aciden Verbindungen ausgewählt aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethylbenzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 1-Methyl-3-phenyl-pyrazolin-5-on, lndan-1,2-dion, Indan-1,3-dion, Indan-1-on, Benzoylacetonitril, 3-Dicyanmethylenindan-1-on, 1,3-Diiminoisoindolin, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinofiniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethytchinoxatinium-p-toluolsulfonat.

Bevorzugte primäre oder sekundäre aromatische Amine der Komponente B sind ausgewählt aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxybenzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)aminoJ-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo )-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie sie in der Formel II dargestellt sind 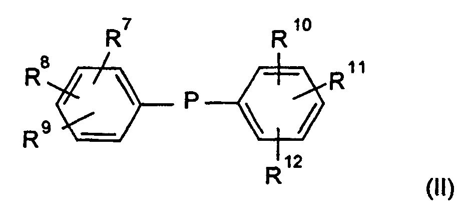 in der
- R⁷ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht,
- R⁸, R⁹, R¹⁰, R¹¹ und R¹² unabhängig voneinander für ein Wasserstoffatom, eine Hydroxy- oder eine Aminogruppe, die durch C₁-C₄-Alkyl-, C₁-C₄-Hydroxyalkyl, C₁-C₄₋Alkoxy-, C₁-C₄-Aminoalkyl- oder C₁-C₄-Alkoxy-C₁-C₄-alkylgruppen substituiert sein kann, stehen, und
- P für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfoxy-, Sulfonyl- oder Iminogruppe, ein Sauerstoff- oder Schwefelatom, oder eine Gruppe mit der Formel III

   -Q'-(CH₂-Q-CH₂-Q")ₒ- (III)

   in der
- Q eine direkte Bindung, eine CH₂- oder CHOH-Gruppe bedeutet,
- Q' und Q" unabhängig voneinander für ein Sauerstoffatom, eine NR¹³-Gruppe, worin R¹³ ein Wasserstoffatom, eine C₁₋₄-Alkyl- oder eine Hydroxy-C₁₋₄-alkylgruppe, wobei auch beide Gruppen zusammen mit dem Restmolekül einen 5-, 6- oder 7-Ring bilden können, bedeutet, die Gruppe O-(CH₂)_{P}-NH oder NH-(CH₂)ₚ-O, worin p und p' 2 oder 3 sind, stehen und
- o eine Zahl von 1 bis 4 bedeutet,
wie beispielsweise 4,4'-Diaminostüben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure.

Die voranstehend genannten Verbindungen mit der Formel I und die Verbindungen der Komponente B werden vorzugsweise in den erfindungsgemäßen Mitteln jeweils in einer Menge von 0,03 bis 65 mmol, insbesondere von 1 bis 40 mmol, bezogen auf 100 g des gesamten Färbemittels, verwendet. Das molare Verhältnis von der Verbindung mit der Formel I und der Verbindung der Komponente B kann im Bereich von 0,5 bis 2,0 liegen, wobei vorzugsweise äquimolare Mengen eingesetzt werden.

Die erfindungsgemäße Kombination kann als direktziehendes Färbemittel oder in Gegenwart von üblichen Oxidationsfarbstoffvorprodukten, wie üblichen Kuppler- und Entwicklerkomponenten, die bereits im einleitenden Teil der Beschreibung genannt wurden, eingesetzt werden.

Färbemittel, die als färbende Komponente die erfindungsgemäße Kombination allein enthalten, werden bevorzugt für Färbungen im gelb-, orange-, rot-, braun- und violett-Bereich bis hin zu schwarz eingesetzt.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, lmidazol, 1-Methylimidazol, Histidin, Pyrrolidin, Prolin, Pyrrolidon, Pyrrolidon-5-carbonsäure, Pyrazol, 1,2,4-Triazol, Piperazin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g des gesamten Färbemittels, eingesetzt werden.

In allen Färbemitteln können auch mehrere verschiedene Kombinationen aus den Verbindungen mit der Formel I und der Komponente B gemeinsam zum Einsatz kommen; ebenso können auch mehrere verschiedene Farbverstärker gemeinsam verwendet werden.

Auf die Anwesenheit von Oxidationsmitteln, z. B. H₂O₂, kann dabei verzichtet werden. Es kann jedoch unter Umständen wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂. Auch Gemische von mehreren Oxidationsmitteln, wie beispielsweise eine Kombination aus Wasserstoffperoxid und Peroxodisulfaten der Alkail- und Erdalkalimetalle oder aus lodidionenquellen, wie z.B. Alkalimetalliodiden und Wasserstoffperoxid oder den vorgenannten Peroxodisulfaten, können verwendet werden. Das Oxidationsmittel bzw. die Oxidationsmittelkombination können erfindungsgemäß in Verbindung mit Oxidationskatalysatoren in dem Haarfärbemittel zur Anwendung kommen. Oxidationskatalysatoren sind beispielsweise Metallsalze oder Metalloxide, die einen leichten Wechsel zwischen zwei Oxidationsstufen der Metallionen ermöglichen. Beispiele sind Salze oder Oxide von Eisen, Ruthenium, Mangan und Kupfer.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red BN, Basic Red 76, HC Blue 2, Disperse Blue 3, Basic Blue 99, HC Violet 1, Disperse Violet 1, Disperse Violet 4, Disperse Black 9 und Basic Brown 16 bekannten Verbindungen sowie 6-Nitro-1,2,3,4-tetrahydrochinoxalin, 4-N-Ethyl-1,4-bis(2-hydroxyethylamino)-2-nitrobenzol-hydrochlorid und 1-Methyl-3-nitro-4-(2-hydroxyethylamino)-benzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, daß die erfindungsgemäß enthaltenen Verbindungen der Formel 1 sowie der Komponente B oder die fakultativ enthaltenen Farbverstärker und direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Citronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈₋Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quaternium-80).

Alkylamidoamine, insbesondere Fettsäureamidoamine wie das unter der Bezeichnung Tego Amid® S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quatemäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homofogenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Aikalimetallaikoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchforid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.
- weitere Oxidationsfarbstoffvorprodukte vom Entwickler und/oder Kupplertyp, wie sie beispielsweise in der WO 01/41716 offenbart werden, auf die hier ausdrücklich Bezug genommen wird

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65 mmol, insbesondere von 1 bis 40 mmol bezogen auf 100 g des gesamten Färbemittels, enthalten.

Der pH-Wert der gebrauchsfertigen Färbezubereitungen liegt üblicherweise zwischen 2 und 12, vorzugsweise zwischen 4 und 10.

Ein zweiter Gegenstand der vorliegenden Erfindung betrifft die Verwendung von einer Kombination aus Dicarbonylverbindungen mit der Formel I 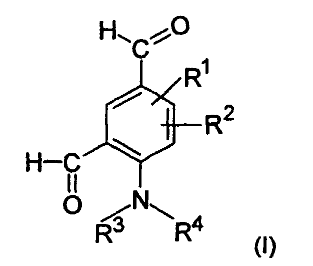 wobei R¹, R², R³ und R⁴ wie oben definiert sind und mindestens einer Verbindung, ausgewählt aus
(a) CH-aciden Verbindungen,
(b) Verbindungen mit primärer oder sekundärer Amino- oder einer Hydroxygruppe, ausgewählt aus aromatischen Hydroxyverbindungen, primären oder sekundären aromatischen Aminen und stickstoffhaltigen heterozyklischen Verbindungen,
und/oder deren physiologisch verträglichen Salzen als färbende Komponente in Färbemitteln, insbesondere Haarfärbemitteln.

Ein dritter Gegenstand der Erfindung ist ein Verfahren zum Färben von keratinhaltigen Fasern, insbesondere menschlichen Haaren, worin ein Färbemittel, enthaltend eine Kombination aus Dicarbonylverbindungen mit der Formel I 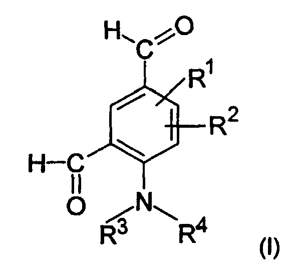 wobei R¹, R², R³ und R⁴ wie oben definiert sind und mindestens eine Verbindung, ausgewählt aus
(a) CH-aciden Verbindungen,
(b) Verbindungen mit primärer oder sekundärer Amino- oder einer Hydroxygruppe, ausgewählt aus aromatischen Hydroxyverbindungen, primären oder sekundären aromatischen Aminen und stickstoffhaltigen heterozyklischen Verbindungen,
und/oder deren physiologisch verträglichen Salzen sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die Dicarbonylverbindungen der Formel I und die Verbindungen der Komponente B können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die Dicarbonylverbindungen der Formel 1 und die Verbindungen der Komponente B können entweder getrennt oder zusammen gelagert werden, entweder in einer flüssigen bis pastösen Zubereitung (wäßrig oder wasserfrei) oder als trockenes Pulver. Bei der getrennten Lagerung werden die Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### Herstellung einer Färbelösung

Es wurde eine Mischung von 3 mmol einer Verbindung der Komponente B und 0,41 g Natriumacetat in 30 g Wasser hergestellt. Unmittelbar vor der Anwendung wurden zu dieser Mischung 3 mmol der Dicarbonylverbindung gemäß Formel I (Komponente A) gegeben und der pH-Wert mit einer 10%igen, wäßrigen NaOH-Lösung auf pH 6 eingestellt.

In diese Färbemischung wurde bei 30°C 30 Minuten lang eine Menschenhaarsträhne (Fa. Kerling, naturweiß) eingebracht. Die Strähne wurde dann 30 Sekunden mit lauwarmem Wasser gespült, mit warmer Luft getrocknet und anschließend ausgekämmt.

Folgende Verbindungen wurden als Komponente A eingesetzt:

### A 4-Dimethylamino-6-methyl-isophthalaldehyd (erfindungsgemäß)

Folgende Verbindungen der Komponente B wurden eingesetzt
- B1: 1,2,3,3-Tetramethylindoliniumiodid
- B2: 1,3-Indandion
- B3: 2-Amino-4-imino-thiazolin Hydrochlorid
- B4: 3-Ethyl-2-methyl-benzothiazoliumiodid
- B5: Benzoylacetonitril
- B6: 2,4,5,6-Tetraaminopyrimidin-2 H₂SO₄
- B7: 1,8-Bis(2,5-diaminophenoxy)-3,6-dioxaoctan-4 HCl
- B8: 4,4'-Diaminodiphenylamin

Die jeweiligen Farbnuancen und Farbtiefen sind in der nachfolgenden Tabelle 1 wiedergegeben.

**Tabelle 1**

| Komponente A | Komponente B | Farbe |
|---|---|---|
| A | B1 | rot-violett |
| A | B2 | orange-rot |
| A | B3 | gelb-orange |
| A | B4 | rot |
| A | B5 | leuchtend orange |
| A | B6 | orange |
| A | B7 | braun |
| A | B8 | schwarz |

## Patentansprüche

1. Mittel zum Färben von keratinhaltigen Fasern, insbesondere menschlichen Haaren, enthaltend als färbende Komponente eine Kombination aus
A. mindestens einer Dicarbonylverbindung mit der Formel I, 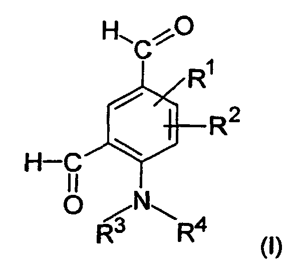 worin
• R¹ und R² stehen unabhängig voneinander für ein Wasserstoffatom, ein Halogenatom, eine C₁-C₆-Alkylgruppe, eine C₂-C₆-Alkenylgruppe, eine C₁-C₆₋Alkoxygruppe, eine C₁-C₆-Hydroxyalkylgruppe, eine C₂-C₆₋Polyhydroxyalkylgruppe, eine Hydroxy-C₁-C₆-alkoxygruppe, eine Hydroxygruppe, eine Sulfonsäuregruppe, eine Carboxygruppe, eine Nitrogruppe, eine Gruppe -NR⁵R⁶, wobei R⁵ und R⁶ stehen unabhängig voneinander für eine C₁-C₆-Alkylgruppe, eine C₂-C₆-Alkenylgruppe, eine C₁₋C₆-Hydroxyalkylgruppe, eine C₁-C₆-Dialkylamino-C₁-C₆-alkylgruppe oder eine Aryl-C₁-C₆-alkylgruppe, oder R⁵ und R⁶ bilden zusammen mit dem Stickstoffatom einen 5-, 6- oder 7-gliedrigen Ring,
oder die Reste R¹ und R² zusammen einen 5-, 6- oder 7-gliedrigen, ankondensierten, aliphatischen oder aromatischen, carbozyklischen oder heterozyklischen Ring bilden,
• R³ und R⁴ stehen unabhängig voneinander für eine C₁-C₆-Alkylgruppe, eine C₂-C₆-Alkenylgruppe, eine C₁-C₆-Hydroxyalkylgruppe, eine C₁-C₆₋Dialkylamino-C₁-C₆-alkylgruppe oder eine Aryl-C₁-C₆-alkylgruppe, oder bilden zusammen mit dem Stickstoffatom einen 5-, 6- oder 7-gliedrigen Ring,
oder deren physiologisch verträglichen Salzen und
B. mindestens einer Verbindung ausgewählt aus
(a) CH-aciden Verbindungen,
(b) Verbindungen mit primärer oder sekundärer Amino- oder einer Hydroxygruppe, ausgewählt aus aromatischen Hydroxyverbindungen, primären oder sekundären aromatischen Aminen und stickstoffhaltigen heterozyklischen Verbindungen,
und/oder deren physiologisch verträglichen Salzen,

2. Mittel nach Anspruch 1, **dadurch gekennzeichnet, daß** die Verbindungen mit der Formel I ausgewählt sind aus 4-Dimethylamino-6-methyl-isophthalaldehyd, 4-Dimethylamino-6-methoxy-isophthalaldehyd und 4-Dimethylamino-isophthalaldehyd.

3. Mittel nach einem der Ansprüche 1 oder 2, **dadurch gekennzeichnet, daß** es als Komponente B mindestens eine CH-acide Verbindung enthält, ausgewählt aus 1,2,3,3-Tetramethyl-3H-indoliumiodid, 1,2,3, 3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 1-Methyl-3-phenyl-pyrazolin-5-on, Indan-1,2-dion, Indan-1,3-dion, Indan-1-on, Benzoylacetonitril, 3-Dicyanmethylenindan-1-on, 1,3-Diiminoisoindolin, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

4. Mittel nach einem der Ansprüche 1 bis 3, **dadurch gekennzeichnet, daß** es als Komponente B mindestens ein primäres oder sekundäres aromatisches Amin enthält, ausgewählt aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxy-benzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan oder deren physiologisch verträglichen Salze.

5. Mittel nach einem der Ansprüche 1 bis 4, **dadurch gekennzeichnet, daß** es als Komponente B mindestens eine stickstoffhaltige heterozyklische Verbindung enthält, ausgewählt aus 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin, 4-Hydroxyindolin und Hydroxypyrimidin-Derivaten sowie die physiologisch verträglichen Salze der vorgenannten Verbindungen.

6. Mittel nach einem der Ansprüche 1 bis 5, **dadurch gekennzeichnet, daß** es als Komponente B mindestens eine aromatische Hydroxyverbindung enthält, ausgewählt aus 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure.

7. Mittel nach einem der Ansprüche 1 bis 6, **dadurch gekennzeichnet, daß** die Dicarbonylverbindungen mit der Formel I und die Verbindungen der Komponente B jeweils in einer Menge von 0,03 bis 65 mmol, insbesondere von 1 bis 40 mmol, bezogen auf 100 g des gesamten Färbemittels, enthalten sind.

8. Mittel nach einem der Ansprüche 1 bis 7, **dadurch gekennzeichnet, daß** die Dicarbonylverbindung mit der Formel I und die Verbindung der Komponente B im molaren Verhältnis von 0,5 bis 2,0 vorliegen.

9. Mittel nach einem der Ansprüche 1 bis 8, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazin oder deren beliebigen Gemischen enthält.

10. Mittel nach einem der Ansprüche 1 bis 9, **dadurch gekennzeichnet, daß** es direktziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

11. Mittel nach einem der Ansprüche 1 bis 10, **dadurch gekennzeichnet, daß** es anionische, zwitterionische oder nichtionische Tenside enthält.

12. Mittel nach einem der Ansprüche 1 bis 11, **dadurch gekennzeichnet, daß** es Oxidationsmittel, insbesondere H₂O₂, in einer Menge von 0,01 bis 6 Gew.-%, bezogen auf die Anwendungslösung, enthält.

13. Mittel nach einem der Ansprüche 1 bis 12, **dadurch gekennzeichnet, daß** es Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, enthält.

14. Verwendung von einer Kombination gemäß Anspruch 1 aus Dicarbonylverbindungen mit der Formel I und CH-aciden Verbindungen und/oder Verbindungen mit einer primären oder sekundären Amino- oder einer Hydroxygruppe, und/oder deren physiologisch verträglichen Salzen als färbende Komponente in Färbemitteln, insbesondere Haarfärbemitteln.

15. Verfahren zum Färben von keratinhaltigen Fasern, insbesondere menschlichen Haaren, worin ein Färbemittel, enthaltend
eine Kombination gemäß Anspruch 1 aus Dicarbonylverbindungen mit der Formel I und CH-aciden Verbindungen und/oder Verbindungen mit einer primären oder sekundären Amino- oder einer Hydroxygruppe, und/oder deren physiologisch verträglichen Salzen, sowie übliche kosmetische Inhaltsstoffe,
auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Revendications

1. Agent pour teindre des fibres kératiniques, en particulier les cheveux humains, contenant en tant que composant colorant, une combinaison de
A. au moins un composé dicarbonyle de formule I, 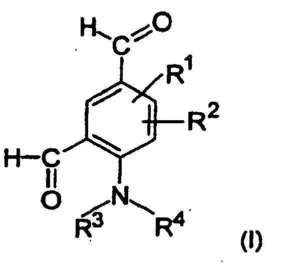 dans laquelle
• R¹ et R² représentent indépendamment l'un de l'autre un atome d'hydrogène, un atome d'halogène, un groupe alkyle en C₁ à C₆, un groupe alcényle en C₂ à C₆, un groupe alcoxy en C₁ à C₆, un groupe hydroxyalkyle en C₁ à C₆, un groupe polyhydroxyalkyle en C₂ à C₆, un groupe hydroxy(alcoxy en C₁ à C₆), un groupe hydroxy, un groupe acide sulfonique, un groupe carboxy, un groupe nitro, un groupe -NR⁵R⁶, R⁵ et R⁶ représentant indépendamment l'un de l'autre un groupe alkyle en C₁ à C₆, un groupe alcényle en C₂ à C₆, un groupe hydroxyalkyle en C₁ à C₆, un groupe (dialkylamino en C₁ à C₆)-(alkyle en C₁ à C₆) ou un groupe aryle(alkyle en C₁ à C₆), ou bien R⁵ et R⁶ forment ensemble avec l'atome d'azote un cycle à 5, 6 ou 7 maillons, ou bien les résidus R¹ et R² ensemble forment un cycle carbocyclique ou hétérocyclique, aliphatique ou aromatique, condensé, à 5, 6 ou 7 maillons,
• R³ et R⁴ représentent indépendamment l'un de l'autre un groupe alkyle en C₁ à C₆, un groupe alcényle en C₂ à C₆, un groupe hydroxyalkyle en C₁ à C₆, un groupe (dialkylamino en C₁ à C₆)-(alkyle en C₁ à C₆) ou un groupe aryle(alkyle en C₁ à C₆) ou forment ensemble avec l'atome d'azote un cycle à 5, 6 ou 7 maillons,
ou ses sels acceptables sur le plan physiologique et
B. au moins un composé choisi parmi
(a) des composés à groupe CH acide,
(b) les composés ayant un groupe amine primaire ou secondaire, ou un groupe hydroxy, choisis parmi les composés hydroxy aromatiques, les amines aromatiques primaires ou secondaires et les composés hétérocycliques azotés,
et/ou ses sels acceptables sur le plan physiologique.

2. Agent selon la revendication 1, **caractérisé en ce que** les composés de formule I sont choisis parmi le 4-diméthylamino-6-méthyl-isophtalaldéhyde, le 4-diméthylamino-6-méthoxyisophtalaldéhyde, et le 4-diméthylaminoisophtalaldéhyde.

3. Agent selon l'une quelconque des revendications 1 ou 2, **caractérisé en ce qu'**il contient en tant que composant B, au moins un composé à groupe CH acide, choisi parmi l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, la 1,3,3-triméthyl-2-méthylèneindoline (base de Fischer), l'iodure de 2,3-diméthylbenzothiazolium, le p-toluènesulfonate de 2,3-diméthylbenzothiazolium, le p-toluènesulfonate de 2,3-diméthylnaphto[1,2-d]thiazolium, le p-toluènesulfonate de 3-éthyl-2-méthylnaptho[1,2-d}thiazolium, la rhodanine, l'acide rhodanine-3-acétique, l'iodure de 1,4-diméthylquinoléinium, l'iodure de 1,2-diméthylquinoléinium, l'acide barbiturique, l'acide thiobarbiturique, l'acide 1,3-diméthylthiobarbiturique, l'acide 1,3-diéthylthiobarbiturique, l'acide 1,3-diéthylbarbiturique, l'oxindole, l'acétate de 3-indoxyle, la 2-coumaranone, la 5-hydroxy-2-coumaranone, la 6-hydroxy-2-coumaranone, la 1-méthyl-3-phénylpyrazolin-5-one, l'indane-1,2-dione, l'indane-1,3-dione, l'indan-1-one, le benzoylacétonitrile, la 3-dicyanométhylèneindan-1-one, la 1,3-diiminoisoindoline, le chlorhydrate de 2-amino-4-imino-1,3-thiazoline, la 5,5-diméthylcyclohexane-1,3-dione, la 2H-1,4-benzoaxazin-4H-3-one, l'iodure de 3-éthyl-2-méthylbenzoxazolium, l'iodure de 3-éthyl-2-méthyl-benzothiazolium, l'iodure de 1-éthyl-4-méthylquinoléinium, l'iodure de 1-éthyl-2-méthylquinoléinium, l'iodure de 1,2,3-triméthylquinoxalinium, le p-toluènesulfonate de 3-éthyl-2-méthylbenzoxazolium, le p-toluènesulfonate de 3-éthyl-2-méthylbenzothiazolium, le p-toluènesulfonate de 1-éthyle-4-méthylquinoléinium, le p-toluènesulfonate de 1-éthyl-2-méthylquinoléinium, et le p-toluènesulfonate de 1,2,3-triméthylquinoxalinium.

4. Agent selon l'une quelconque des revendications 1 à 3, **caractérisé en ce qu'**il contient en tant que composant B, au moins une amine aromatique primaire ou secondaire choisie parmi la N,N=diméthyl-p-phénylènediamine, la N,N-diéthyl-p-phénylènediamine, la N-(2-hydroxyéthyl)-N-éthyl-p-phénylènediamine, la N,N-bis-(2-hydroxyéthyl)-p-phénylènediamine, la N-(2-méthoxyéthyl)-p-phénylènediamine, la 2,3-dichloro-p-phénylènediamine, la 2,4-dichloro-p-phénylènediamine, la 2,5-dichloro-p-phénylènediamine, la 2-chloro-p-phénylènediamine, la 2,5-dihydroxy-4-morphollinoaniline, le 2-aminophénol, le 3-aminophénol, le 4-aminophénol, le 2-aminométhyl-4-aminophénol, le 2-hydroxyméthyl-4-aminophénol, l'o-phénylènediamine, la m-phénylènediamine, la p-phénylènediamine, le 2,5-diaminotoluène, .le 2,5-diaminophénol, le 2,5-diaminoanisole, le 2,5-diaminophénétol, le 4-amino-3-méthylphénol, le 2-(2,5-diaminophényl)éthanol, le 2,4-diaminophénoxyéthanol, le 2(2,5-diaminophénoxy)éthanol, le 3-amino-4-(2-hydroxyéthyloxy)phénol, le 3,4-méthylènedioxyphénol, la 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-(2-hydroxyéthylamino)phénol, le 3-amino-2-chloro-6-méthylphénol, le 2-méthyl-5-amino-4-chlorophénol, le 5-(2-hydroxyéthylamino)=4-méthoxy-2-méthylphénol, le 4-amino-2-hydroxyméthylphénol, le 2-(diéthylaminométhyl)-4-aminophénol, le 4-amino-1-hydroxy-2-(2₋hydroxyéthylaminométhyl)benzène, le 1-hydroxy-2-amino-5-méthylbènzène, le 1-hydroxy-2-amino-6-méthylbenzène, le 2-amino-5-acétamidophénol, le 1,3-diméthyl-2,5-diaminobenzène, le 5-(3-hydroxypropylamino)-2-méthylphénol, le 5-amino-4-méthoxy-2-méthylphénol, le N,N-diméthyl-3-aminophénol, le N-cyclopentyl-3-aminophénol, le 5-amino-4-fluoro-2-méthylphénol, le 2,4-diamino-5-flurotoluène, le 2,4-diamino-5-(2-hydroxyéthoxy)toluène, le 2,4-diamino-5-méthylphénétol, le 3,5-diamino-2-méthoxy-1-méthylbenzène, le 2-amino-4-(2-hydroxyéthylamino)anisole, le 2,6-bis-(2-hydroxyéthylamino)-1-méthylbenzène, le 1,3-diamino-2,4-diméthoxybenzène, le 3,5-diamino-2-méthoxytoluène, l'acide 2-aminobenzoïque, l'acide 3-aminobenzoïque, l'acide 4-aminobenzoïque, l'acide 2-aminophénylacétique, l'acide 3-aminophénylacétique, l'acide 4-aminophénylacétique, l'acide 2,3-diaminobenzoïque, l'acide 2,4-diaminobenzoïque, l'acide 2,5-diaminobenzoïque, l'acide 3,4-diaminobenzoïque, l'acide 3,5-diaminobenzoïque, l'acide 4-aminosalicylique, l'acide 5-aminosalicylique, l'acide 3-amino-4-hydroxybenzoïque, l'acide 4-amino-3-hydroxybenzoïque, l'acide 2-aminobenzènesulfonique, l'acide 3-aminobenzènesulfonique, l'acide 4-aminobenzènesulfonique, l'acide 3-amino-4-hydroxybenzènesulfonique, l'acide 4-amino-3-hydroxynapthalène-1-sulfonique, l'acide 6-amino-7-hydroXynaphtalène-2-sulfonique, l'acide 7-amino-4-hydroxynapthtalène-2-sulfonique, l'acide 4-amino-5-hydroxynapthalène-2,7-disulfonique, l'acide 3-amino-2-napthoïque, l'acide 3-aminophtalique, l'acide 5-aminoisophtalique, le 1,3,5-triaminobenzène, le 1,2,4-triaminobenzène, le 1,2,4,5-tétraaminobenzène, le 2,4,5-triaminophénol, le pentaaminobenzène, l'hexaaminobenzène, la 2,4,6-triaminorésorcine, la 4,5-diaminopyrocatéchine, le 4,6-diaminopyrogallol, la 1-(2-hydroxy-5-aminobenzyle)-2-imidazolidinone, le 4-amino-2-((4-[(5-amino-2-hydroxyphényl)méthyl]pipérazinyl)méthyl)phénol, la 3,5-diamino-4-hydroxypyrrocatéchine, le 1,4-bis-(4-aminophényl)-1,4-diazacyctoheptane, les nitriles aromatiques, comme le 2-amino-4-hydroxybenzonitrile, le 4-amino-2-hydroxybenzonitrile, le 4-aminobenzonitrile, le 2,4-diaminobenzonitrile, les composés amino contenant des groupes nitro, comme la 3-amino-6-méthylamino-2-nitropyridine, l'acide picramique, le chlorure de [8-[(4-amino-2-nitrophényl)-azo]-7-hydroxynapht-2-yl]triméthylammonium, le chlorure de [8-[(4-amino-3-nitrophényl)-azo]-7-hydroxy-napht-2-yl]triméthylammonium (Basic Brown 17), le 1-hydroxy-2-amino-4,6-dinitrobenzène, le 1-amino-2-nitro-4[bis-(2-hydroxyéthyl)amino]benzène, le 1-amino-2-[(2-hydroxyéthyl)amino]-5-nitrobenzène (HC Yellow N°5), le 1-amino-2-nitro-4-[(2-hydroxyéthyl)amino]benzène (HC Red N°7), la 2-chloro-5-nitro-N-2-hydroxyéthyl-1,4-phénylènediamine, le 1-[(2-hydroxyéthyl)amino]-2-nitro-4-aminobenzène (HC Red N°3), le 4-amino-3-nitrophénol, le 4-amino-2-nitrophénol, la 6-nitro-o-toluidine, le 1-amino-3-méthyl-4-[(2-hydroxyéthyl)-amino]-6-nitrobenzène (HC Violet . N)1), le 1-amino-2-nitro-4-[(2,3-dihydroxypropyl)amino]-5-chlorobenzène (HC Red N°10), l'acide 2-(4-amino-2-nitroanilino)benzoïque, la 6-nitro-2,5-diaminopyridine, le 2-amino-6-chloro-4-nitrophénol, le sel disodique de l'acide 1-amino-2-(3-nitrophénylazo)-7-phénylazo-8-napthol-3,6-disulfonique (Acid Blue N°29), le sel disodique de l'acide 1-amino-2-(2-hydroxy-4-nitrophénylazo)-8-napthol-3,6-disulfonique (Palatinchrome green), le sel disodique de l'acide 1-amino-2-(3-chloro-2-hydroxy-5-nitrophénylazo)-8-napthol-3,6-disulfonique (Gallion), le sel sodique de l'acide 4-amino-4'-nitrostilbène-2,2'-disulfonique, le 2,4-diamino-3',5'-dinitro-2'-hydroxy-5-méthyl-azobenzène (Mordant Brown 4), l'acide 4'-amino-4-nitro-diphénylamine-2-sulfonique, l'acide 4'-amino-3'-nitrobenzophénone-2-carboxylique, le 1-amino-4-nitro-2-(2-nitrobenzylidèneamino)benzène, la 2-[2-(diéthylamino)éthylamino]-5-nitroaniline, l'acide 3-amino-4-hydroxy-5-nitrobenzènesulfonique, le 3-amino-3'-nitrobiphényle, le 3-amino-4-nitroacénapthène, la 2-amino-1-nitronaphtalène, le 5-amino-6-nitrobenzo-1,3-dioxole, les anilines, en particulier les anilines contenant des groupes nitro, comme la 4-nitroaniline, la 2-nitroaniline, le 1,4-diamino-2-nitrobenzène, le 1,2-diamino-4-nitrobenzène, le 1-amino-2-méthyl-6-nitrobenzène, la 4-nitro-1,3-phénylènediamine, le 2-nitro-4-amino-1-(2-hydroxyéthylamino)benzène, le 2-nitro-1-amino-4-[bis-(2hydroxyéthyl)amino]benzène, l'acide 4-amino-2-nitrodiphénylamine-2'-carboxylique, le 1-amino-5-chloro-4-(2-hydroxyéthyl-amino)-2-nitrobenzène, le 4,4'-diaminostylbène et son chlorhydrate, le sel monosodique ou disodique de l'acide 4,4'-diaminostylbène-2,2'-disulfonique, le 4-amino-4'-diméthylaminostylbène et son chlorhydrate, le 4,4'-diaminodiphénylméthane, le sulfure de 4,4'-diaminodiphényle, le 4,4'-diaminodiphénylsulfoxyde, la 4,4'-diaminodiphénylamine, l'acide 4,4'-diaminodiphénylamine-2-sulfonique, la 4,4'-diaminobenzophénone, l'éther 4,4'-diaminodiphénylique, le 3,3',4,4'-tétraaminodiphényle, la 3,3',4,4'-tétraaminobenzophénone, le 1,3-bis-(2,4-diaminophénoxy)propane, le 1,8-bis-(2,5-diaminophénoxy)-3,6-dioxyoctane, le 1,3-bis-(4-aminophényl)aminopropane, le 1,3-bis-(4-aminophénylamino)-2-propanol, le 1,3-bis-[N-(4-aminophényl)-2-hydroxyéthylamino]-2-propanol, la N,N-bis-[2-(4-aminophénoxy)-éthyl]-méthylamine, la N-phényl-1,4-phénylènediamine et le bis-(5-amino-2-hydroxyphényl)méthane ou leurs sels acceptables sur le plan physiologique.

5. Agent selon l'une quelconque des revendications 1 à 4, **caractérisé en ce qu'**il contient en tant que composant B, au moins un composé hétérocyclique azoté, choisi parmi la 2-aminopyridine, la 3-aminopyridine, la 4-aminopyridine, la 2-amino-3-hydroxypyridine, la 2,6-diaminopyridine, la 2,5-diaminopyridine, la 2-(aminoéthylamino)-5-aminopyridine, la 2,3-diaminopyridine, la 2-diméthylamino-5-aminopyridine, la 2-méthylamino-3-amino-6-méthoxypyridine, la 2,3-diamino-6-méthoxypyridine, la 2,6-diméthoxy-3,5-diaminopyridine, la 2,4,5-triaminopyridine, la 2,6-dihydroxy-3,4-diméthylpyridine, la N-[2-(2,4-diaminophényl)aminoéthyl]-N-(5-amino-2-pyridyl)amine, la N-[2-(4-aminophényl)aminoéthyl]-N-(5-amino-2-pyridyl)amine, la 2,4-dihydroxy-5,6-diaminopyrimidine, la 4,5,6-triaminopyrimidine, la 4-hydroxy-2,5,6-triaminopyrimidine, la 2-hydroxy-4,5,6-triaminopyrimidine, la 2,4,5,6-tétraaminopyrimidine, la 2-méthylamino-4,5,6-triaminopyrimidine, la 2,4-diaminopyrimidine, la 4,5-diaminopyrimidine, la 2-amino-4-méthoxy-6-méthylpyrimidine, le 3,5-diaminopyrazole, le 3,5-diamino-1,2,4-triazole, le 3-aminopyrazole, le 3-amino-5-hydroxypyrazole, le 1-phényl-4,5-diaminopyrazole, le 1-(2-hydroxyéthyl)-4,5-diaminopyrazole, le 1-phényl-3-méthyl-4,5-diaminopyrazole, la 4-amino-2,3-diméthyl-1-phényl-3-pyrazolin-5-one (la 4-aminoantipyrine), la 1-phényl-3-méthylpyrazol-5-one, la 2-aminoquinoléine, la 3-aminoquinoléine, la 8-aminoquinoléine, la 4-aminoquinaldine, l'acide 2-aminonicotinique, l'acide 6-aminonicotinique, la 5-aminoisoquinoléine, le 5-aminoindazole, le 6-aminoindazole, le 5-aminobenzimidazole, le 7-aminobenzimidazole, le 5-aminobenzothiazole, le 7-aminobenzothiazole, la 2,5-dihydroxy-4-morpholinoaniline, ainsi que les dérivés d'indole et d'indoline, comme le 4-aminoindole, le 5-aminoindole, le 6-aminoindole, le 7-aminoindole, le 5,6-dihydroxyindole, la 5,6-dihydroxyindoline, la 4-hydroxyindoline, et les dérivés d'hydroxypyrimidine, ainsi que les sels acceptables sur le plan physiologique des composés précités.

6. Agent selon l'une quelconque des revendications 1 à 5, **caractérisé en ce qu'**il contient en tant que composant B, au moins un composé hydroxy aromatique, choisi parmi la 2-méthylrésorcine, la 4-méthylrésorcine, la 5-méthylrésorcine, la 2,5-diméthylrésorcine, la résorcine, le 3-méthoxyphénol, la pyrocatéchine, l'hydroquinone, le pyrogallol, la phloroglucine, l'hydroxyhydroquinone, le 2-méthoxyphénol, le 3-méthoxyphénol, le 4-méthoxyphénol, le 3-diméthylaminophénol, le 2-(2-hydroxyéthyl)phénol, le 3,4-méthylènedioxyphénol, l'acide. 2,4-dihydroxybenzoïque, l'acide 3,4-dihydroxybenzoïque, l'acide 2,4-dihydroxyphénylacétique, l'acide 3,4-dihydroxyphénylacétique, l'acide gallique, l'acide 2,4,6-trihydroxybenzoïque, la 2,4,6-trihydroxyacétophénone, la 2-chlororésorcine, la 4-chlororésorcine, le 1-naphtol, le 1,5-dihydroxynaphtalène, le 2,3-dihydroxynaphtalène, le 2,7-dihydroxynapthalène, l'acide 6-diméthylamino-4-hydroxy-2-naphtalène-sulfonique, et l'acide 3,6-dihydroxy-2,7-naphtalènesulfonique.

7. Agent selon l'une quelconque des revendications 1 à 6, **caractérisé en ce que** les composés dicarbonyle ayant la formule I et les composés du composant B sont contenus respectivement en une quantité de 0,03 à 65 mmoles, en particulier de 1 à 40 mmoles, par rapport à 100 g de la teinture totale.

8. Agent selon l'une quelconque des revendications 1 à 7, **caractérisé en ce que** le composé dicarbonyle ayant la formule 1 et le composé du composant B existent dans un rapport molaire de 0,5 à 2,0.

9. Agent selon l'une quelconque des revendications 1 à 8, **caractérisé en ce qu'**il contient un renforçateur de couleur choisi dans le groupe consistant en la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'histidine, la pyrrolidine, la proline, la pyrrolidone, l'acide pyrrolidone-5-carboxylique, le pyrazole, le 1,2,4-triazole, la pipérazine ou leurs mélanges quelconques.

10. Agent selon l'une quelconque des revendications 1 à 9, **caractérisé en ce qu'**il contient des colorants montant directement sur les fibres issus du groupe des nitrophénylènediamines, des nitroaminophénols, des anthraquinones, ou des indophénols, de préférence en une quantité de 0,01 à 20% en poids, par rapport à la teinture totale.

11. Agent selon l'une quelconque des revendications 1 à 10, **caractérisé en ce qu'**il contient des tensio-actifs anioniques, zwitterioniques ou non ioniques.

12. Agent selon l'une quelconque des revendications 1 à 11, **caractérisé en ce qu'**il contient des agents d'oxydation, en particulier H₂O₂, en une quantité de 0,01 à 6% en poids par rapport à la solution d'application.

13. Agent selon l'une quelconque des revendications 1 à 12, **caractérisé en ce qu'**il contient des sels d'ammonium ou des sels métalliques choisis dans le groupe formé par les formiates, les carbonates, les halogénures, les sulfates, les butyrates, les valériates, les capronates, les acétates, les lactates, les glycolates, les tartrates, les citrates, les gluconates, les propionates, les phosphates et les phosphonates de métaux alcalins, comme le potassium, le sodium ou le lithium, de métaux alcalino-terreux comme le magnésium, le calcium, le strontium ou le baryum, ou de l'aluminium, du manganèse, du fer, du cobalt, du cuivre ou du zinc.

14. Utilisation d'une combinaison selon la revendication 1 de composés dicarbonyle ayant la formule I et de composés à groupe CH acide et/ou de composés ayant un groupe amine primaire ou secondaire ou un groupe hydroxy, et/ou de leurs sels acceptables sur le plan physiologique en tant que composant colorant dans les teintures, en particulier les teintures pour cheveux.

15. Procédé de teinture de fibres kératiniques, en particulier les cheveux humains, dans lequel une teinture, contenant
une combinaison selon la revendication 1 de composés dicarbonyle ayant la formule I et de composés à groupe CH acide, et/ou de composés ayant un groupe amine primaire ou secondaire, ou un groupe hydroxy, et/ou de leurs sels acceptables sur le plan physiologique, ainsi que d'ingrédients cosmétiques courants, est appliquée sur les fibres kératiniques, et laissée pendant quelques temps, couramment environ 30 minutes, sur la fibre, puis est rincée à nouveau ou éliminée par lavage avec un shampoing.

## Claims

1. Agent for dyeing keratinous fibres, in particular human hair, comprising, as dyeing component, a combination of
A. at least one dicarbonyl compound with the formula I, 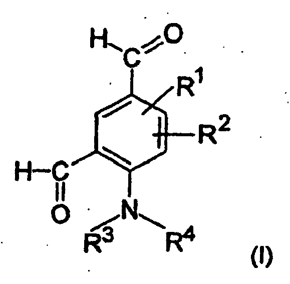 in which
• R¹ and R², independently of one another, are a hydrogen atom, a halogen atom, a C₁-C₆-alkyl group, a C₂-C₆-alkenyl group, a C₁-C₆-alkoxy group, a C₁-C₆-hydroxyalkyl group, a C₂-C₆₋polyhydroxyalkyl group, a hydroxy-C₁-C₆-alkoxy group, a hydroxyl group, a sulphonic acid group, a carboxy group, a nitro group, a group -NR⁵R⁶, where R⁵ and R⁶, independently of one another, are a C₁-C₆-alkyl group, a C₂-C₆₋alkenyl group, a C₁-C₆-hydroxyalkyl group, a C₁-C₆-dialkylamino-C₁-C₆-alkyl group or an aryl-C₁-C₆-alkyl group, or R⁵ and R⁶, together with the nitrogen atom, form a 5-, 6- or 7-membered ring, or the radicals R¹ and R² together form a 5-, 6- or 7-membered, fused-on, aliphatic or aromatic, carbocyclic or heterocyclic ring,
• R³ and R⁴, independently of one another, are a C₁-C₆-alkyl group, a C₂-C₆-alkenyl group, a C₁-C₆-hydroxyalkyl group, a C₁-C₆-dialkylamino-C₁-C₆-alkyl group or an aryl-C₁-C₆-alkyl group, or together with the nitrogen atom form a 5-, 6- or 7-membered ring,
or physiologically compatible salts thereof and
B. at least one compound chosen from
(a) CH-acidic compounds,
(b) compounds with primary or secondary amino group or a hydroxy group chosen from aromatic hydroxy compounds, primary or secondary aromatic amines and nitrogen-containing heterocyclic compounds,
and/or physiologically compatible salts thereof.

2. Agent according to Claim 1, **characterized in that** the compounds with the formula I are chosen from 9-dimethylamino-6-methylisophthaladehyde, 4-dimethylamino-6-methoxyisophthalaldehyde and 4-dimethylaminoisophthalaldehyde.

3. Agent according to one of Claims 1 or 2, **characterized in that** it comprises as component B at least one CH-acidic compound chosen from 1,2,3,3-tetramethyl-3H-indolium iodide, 1,2,3,3-tetramethyl-3H-indolium p-toluenesulphonate, 1,2,3,3-tetramethyl-3H-indolium methanesulphonate, 1,3,3-trimethyl-2-methyleneindoline (Fischer's base), 2,3-dimethylbenzothiazolium iodide, 2,3-dimethylbenzothiazolium p-toluenesulphonate, 2,3-dimethylnaphthol[1,2-d]thiazolium p-toluenesulphonate, 3-ethyl-2-methylnaphthol-[1,2-d]thiazolium p-toluenesulphonate, rhodanine, rhodanine-3-acetic acid, 1,4-dimethylquinolinium iodide, 1,2-dimethylquinolinium iodide, barbituric acid, thiobarbituric acid, 1,3-dimethylthiobarbituric acid, 1,3-diethylthiobarbituric acid, 1,3-diethylbarbituric acid, oxindole, 3-indoxyl acetate, 2-coumaranone, 5-hydroxy-2-coumaranone, 6-hydroxy-2-coumaranone, 1-methyl-3-phenylpyrazolin-5-one, indane-1,2-dione, indane-1,3-dione, indan-1-one, benzoylacetonitrile, 3-dicyanomethyleneindan-1-one, 1,3-diiminoisoindoline, 2-amino-4-imino-1,3-thiazoline hydrochloride, 5,5-dimethylcyclohexane-1,3-dione, 2H-1,4-benzoxazin-4H-3-one, 3-ethyl-2-methylbenzoxazolium iodide, 3-ethyl-2-methylbenzothiazolium iodide, 1-ethyl-4-methylquinolinium iodide, 1-ethyl-2-methylquinolinium iodide, 1,2,3-trimethylquinoxalinium iodide 3-ethyl-2-methylbenzoxazolium p-toluenesulphonate, 3-ethyl-2-methylbenzothiazolium p-toluenesulphonate, 1-ethyl-4-methylquinolinium p-toluenesulphonate, 1-ethyl-2-methylquinolinium p-toluenesulphonate, and 1,2,3-trimethylquinoxalinium p-toluenesulphonate.

4. Agent according to one of Claims 1 to 3, **characterized in that** it comprises, as component B, at least one primary or secondary aromatic amine chosen from N,N-dimethyl-p-phenylenediamine, N,N-diethyl-p-phenylenediamine, N-(2-hydroxyethyl)-N-ethyl-p-phenylenediamine, N,N-bis (2-hydroxyethyl)-p-phenylenediamine, N-(2-methoxyethyl)-p-phenylenediamine, 2,3-dichloro-p-phenylenediamine, 2,4-dichloro-p-phenylenediamine, 2,5-dichloro-p-phenylenediamine, 2-chloro-p-phenylenediamine, 2,5-dihydroxy-4-morpholinoaniline, 2-aminophenol, 3-aminophenol, 4-aminophenol, 2-aminomethyl-4-aminophenol, 2-hydroxymethyl-4-aminophenol, o-phenylenediamine, m-phenylenediamine, p-phenylenediamine, 2,5-diaminotoluene, 2,5-diaminophenol, 2,5-diaminoanisole, 2,5-diaminophenetol, 4-amino-3-methylphenol, 2-(2,5-diaminophenyl)ethanol, 2,4-diaminophenoxyethanol, 2-(2,5-diaminophenoxy)-ethanol, 3-amino-4-(2-hydroxyethyloxy)phenol, 3,4-methylenedioxyphenol, 3,4-methylenedioxyaniline, 3-amino-2,4-dichlorophenol, 4-methylaminophenol, 2-methyl-5-aminophenol, 3-methyl-4-aminophenol, 2-methyl-5-(2-hydroxyethylamino)phenol, 3-amino-2-chloro-6-methylphenol, 2-methyl-5-amino-4-chlorophenol, 5-(2-hydroxyethylamino)-4-methoxy-2-methylphenol, 9-amino-2-hydroxymethylphenol, 2-(diethylaminomethyl)-4-aminophenol, 4-amino-1-hydroxy-2-(2-hydroxyethylaminomethyl)benzene, 1-hydroxy-2-amino-5-methylbenzene, 1-hydroxy-2-amino-6-methylbenzene, 2-amino-5-acetamidophenol, 1,3-dimethyl-2,5-diaminobenzene, 5-(3-hydroxypropylamino)-2-methylphenol, 5-amino-4-methoxy-2-methylphenol, N,N-dimethyl-3-aminophenol, N-cyclopentyl-3-aminophenol, 5-amino-4-fluoro-2-methylphenol, 2,4-diamino-5-fluorotoluene, 2,4-diamino-5-(2-hydroxyethoxy)toluene, 2,4-diamino-5-methylphenetol, 3,5-diamino-2-methoxy-1-methylbenzene, 2-amino-4-(2-hydroxyethylamino)anisole, 2,6-bis(2-hydroxyethylamino)-1-methylbenzene, 1,3-diamino-2,4-dimethoxybenzene, 3,5-diamino-2-methoxytoluene, 2-aminobenzoic acid, 3-aminobenzoic acid, 4-aminobenzoic acid, 2-aminophenylacetic acid, 3-aminophenylacetic acid, 4-aminophenylacetic acid, 2,3-diaminobenzoic acid, 2,4-diaminobenzoic acid, 2,5-diaminobenzoic acid, 3,4-diaminobenzoic acid, 3,5-diaminobenzoic acid, 4-aminosalicylic acid, 5-aminosalicylic acid, 3-amino-4-hydroxybenzoic acid, 4-amino-3-hydroxybenzoic acid, 2-aminobenzenesulphonic acid, 3-aminobenzenesulphonic acid; 4-aminobenzenesulphonic acid, 3-amino-4-hydroxybenzenesulphonic acid, 4-amino-3-hydroxynaphthalene-1-sulphonic acid, 6-amino-7-hydroxynaphthalene-2-sulphonic acid, 7-amino-4-hydroxynaphthalene-2-sulphonic acid, 4-amino-5-hydroxynaphthalene-2,7-disulphonic acid, 3-amino-2-naphthoic acid, 3-aminophthalic acid, 5-aminoisophthalic acid, 1,3,5-triaminobenzene, 1,2,4-triaminobenzene, 1,2,4,5-tetraaminobenzene, 2,4,5-triaminophenol, pentaaminobenzene, hexa-aminobenzene, 2,4,6-triaminoresorcinol, 4,5-diaminopyrocatechin, 4,6-diaminopyrogallol, 1-(2-hydroxy-5-aminobenzyl)-2-imidazolidinone, 9-amino-2-((4-[(5-amino-2-hydroxyphenyl)methyl]piperazinyl) methyl)phenol, 3,5-diamino-4-hydroxypyrocatechin, 1,4-bis(4-aminophenyl)-1,4-diazacycloheptane, aromatic nitriles, such as 2-amino-4-hydroxybenzonitrile, 4-amino-2-hydroxybenzonitrile, 4-aminobenzonitrile, 2,4-diaminobenzonitrile, nitro-group-containing amino compounds, such as 3-amino-6-methylamino-2-nitropyridine, picramic acid, [8-[(4-amino-2-nitrophenyl)azo]-7-hydroxynaphth-2-yl]trimethylammonium chloride, [8-((4-amino-3-nitrophenyl)azo)-7-hydroxynaphth-2-yl]trimethylammonium chloride (Basic Brown 17), 1-hydroxy-2-amino-4, 6-dinitrobenzene, 1-amino-2-nitro-4-[bis(2-hydroxyethyl)amino]benzene, 1-amino-2-[(2-hydroxyethyl)amino]-5-nitrobenzene (HC Yellow No. 5), 1-amino-2-nitro-4-[(2-hydroxyethyl)amino]benzene (HC Red No. 7), 2-chloro-5-nitro-N-2-hydroxyethyl-1,4-phenylenediamine, 1-[(2-hydroxyethyl)amino]-2-nitro-4-aminobenzene (HC Red No. 3), 4-amino-3-nitrophenol, 4-amino-2-nitrophenol, 6-nitro-o-toluidine, 1-amino-3-methyl-4-[(2-hydroxyethyl)amino]-6-nitrobenzene (HC Violet No. 1), 1-amino-2-nitro-4-[(2,3-dihydroxypropyl)amino]-5-chlorobenzene (HC Red No. 10), 2-(4-amino-2-nitroanilino)benzoic acid, 6-nitro-2,5-diaminopyridine, 2-amino-6-chloro-4-nitrophenol, 1-amino-2-(3-nitrophenylazo)-7-phenylazo-8-naphthol-3,6-disulphonic acid disodium salt (Acid Blue No. 29), 1-amino-2-(2-hydroxy-4-nitrophenylazo)-8-naphthol-3,6-disulphonic acid disodium salt (Palatine Chrome Green), 1-amino-2-(3-chloro-2-hydroxy-5-nitrophenylazo)-8-naphthol-3,6-disulphonic acid disodium salt (Gallion), 4-amino-4'-pitrostilbene-2,2'-disulphonic acid disodium salt, 2,4-diamino-3',5'-dinitro-2'-hydroxy-5-methylazobenzene (Mordant Brown 4), 4'-amino-4-nitrodiphenylamine-2-sulphonic acid, 4'-amino-3'-nitrobenzophenone-2-carboxylic acid, 1-amino-9-nitro-2-(2-nitrobenzylidene-amino)benzene, 2-[2-(diethylamino)ethylamino]-5-nitroaniline, 3-amino-4-hydroxy-5-nitrobenzene-sulphonic acid, 3-amino-3'-nitrobiphenyl, 3-amino-9-nitroacenaphthene, 2-amino-1-nitronaphthalene, 5-amino-6-nitrobenzo-1,3-dioxole, anilines, in particular nitro-group-containing anilines, such as 4-nitroaniline, 2-nitroaniline, 1,4-diamino-2-nitrobenzene, 1,2-diamino-4-nitrobenzene, 1-amino-2-methyl-6-nitrobenzene, 4-nitro-1,3-phenylenediamine, 2-nitro-4-amino-1-(2-hydroxyethylamino)-benzene, 2-nitro-1-amino-4-[bis(2-hydroxyethyl)amino]benzene, 4-amino-2-nitrodiphenylamine-2'-carboxylic acid, 1-amino-5-chloro-4-(2-hydroxy-ethylamino)-2-nitrobenzene, 4,4'-diaminostilbene and its hydrochloride, 4,4'-diaminostilbene-2,2'-disulphonic acid mono- or di-Na salt, 4-amino-4'-dimethylaminostilbene and its hydrochloride, 4,4'-diaminodiphenylmethane, 4,4'-diaminodiphenyl sulphide, 4,4'-diaminodiphenyl sulphoxide, 4,4'-diaminodiphenylamine, 4,4'-diaminodiphenylamine-2-sulphonic acid, 4,4'-diaminobenzophenone, 4,4'-diaminodiphenyl ether; 3,3',4,4'-tetraamino-diphenyl, 3,3',4,4'-tetraaminobenzophenone, 1,3-bis(2,4-diaminophenoxy)propane, 1,8-bis(2,5-diaminophenoxy)-3,6-dioxaoctane, 1,3-bis(4-amino-phenylamino)propane, 1,3-bis(4-aminophenylamino)-2-propanol, 1,3-bis[N-(4-aminophenyl)-2-hydroxy-ethylamino]-2-propanol, N,N-bis[2-(4-aminophenoxy)ethyl]methylamine, N-phenyl-1,4 -phenylene-diamine and bis(5-amino-2-hydroxyphenyl)methane, or physiologically compatible salts thereof.

5. Agent according to one of Claims 1 to 4, **characterized in that** it comprises, as component B, at least one nitrogen-containing heterocyclic compound chosen from 2-aminopyridine, 3-aminopyridine, 4-aminopyridine, 2-amino-3-hydroxypyridine, 2,6-diaminopyridine, 2,5-diaminopyridine, 2-(aminoethylamino)-5-aminopyridine, 2,3-diaminopyridine, 2-dimethylamino-5-aminopyridine, 2-methylamino-3-amino-6-methoxypyridine, 2,3-diamino-6-methoxypyridine, 2,6-dimethoxy-3,5-diaminopyridine, 2,4,5-triaminopyridine, 2,6-dihydroxy-3,4-dimethylpyridine, N-[2-(2,4-diaminophenylamino)ethyl)-N-(5-amino-2-pyridyl)amine, N-[2-(4-aminophenyl)aminoethyl]-N-(5-amino-2-pyridyl)amine, 2,4-dihydroxy-5,6-diaminopyrimidine, 4,5,6-triaminopyrimidine, 4-hydroxy-2,5,6-triaminopyrimidine, 2-hydroxy-4,5,6-triaminopyrimidine, 2,4,5,6-tetraaminopyrimidine, 2-methylamino-4, 5, 6-triaminopyrimidine, 2,4-diaminopyrimidine, 4,5-diaminopyrimidine, 2-amino-4-methoxy-6-methylpyrimidine, 3,5-diaminopyrazole, 3,5-diamino-1,2,4-triazole, 3-aminopyrazole, 3-amino-5-hydroxypyrazole, 1-phenyl-4,5-diaminopyrazole, 1-(2-hydroxyethyl)-4,5-diaminopyrazole, 1-phenyl-3-methyl-4,5-diaminopyrazole, 4-amino-2,3-dimethyl-1-phenyl-3-pyrazolin-5-one (4-aminoantipyrine), 1-phenyl-3-methypyrazol-5-one, 2-aminoquinoline, 3-aminoquinoline, 8-aminoquinoline, 4-aminoquinaldine, 2-aminonicotinic acid, 6-aminonicotinic acid, 5-aminoisoquinoline, 5-aminoindazole, 6-aminoindazole, 5-aminobenzimidazole, 7-amino-benzimidazole, 5-aminobenzothiazole, 7-aminobenzothiazole, 2,5-dihydroxy-4-morpholinoaniline, and indole and indoline derivatives, such as 4-aminoindole, 5-aminoindole, 6-aminoindole, 7-aminoindole, 5,6-dihydroxyindole, 5,6-dihydroxy-indoline, 4-hydroxyindoline and hydroxypyrimidine derivatives, and the physiologically compatible salts of the abovementioned compounds.

6. Agent according to one of Claims 1 to 5, **characterized in that** it comprises, as component B, at least one aromatic hydroxy compound chosen from 2-methylresorcinol, 4-methylresorcinol, 5-methylresorcinol, 2,5-dimethylresorcinol, resorcinol, 3-methoxyphenol, pyrocatechin, hydroquinone, pyrogallol, phloroglucinol, hydroxyhydroquinone, 2-methoxyphenol, 3-methoxyphenol, 4-methoxyphenol, 3-dimethylaminophenol, 2-(2-hydroxyethyl) phenol, 3,9-methylenedioxyphenol, 2,4-dihydroxybenzoic acid, 3,4-dihydroxybenzoic acid, 2,4-dihydroxyphenylacetic acid, 3,4-dihydroxyphenylacetic acid, gallic acid, 2,4,6-trihydroxybenzoic acid, 2,4,6-trihydroxy-acetophenone, 2-chlororesorcinol, 4-chlororesorcinol, 1-naphthol, 1,5-dihydroxynaphthalene, 2,3-dihydroxynaphthalene, 2,7-dihydroxynaphthalene, 6-dimethylamino-4-hydroxy-2-naphthalenesulphonic acid and 3,6-dihydroxy-2,7-naphthalenesulphonic acid.

7. Agent according to one of Claims 1 to 6, **characterized in that** the dicarbonyl compounds with the formula I and the compounds of component B are each present in an amount of from 0.03 to 65 mmol, in particular from 1 to 40 mmol, based on 100 g of the total dyeing agent.

8. Agent according to one of Claims 1 to 7, **characterized in that** the dicarbonyl compound with the formula I and the compound of component B are present in the molar ratio from 0.5 to 2.0.

9. Agent according to one of Claims 1 to 8, **characterized in that** it comprises colour enhancers chosen from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, histidine, pyrrolidine, proline, pyrrolidone, pyrrolidone-5-carboxylic acid, pyrazole, 1,2,4-triazole, piperazine or any mixtures thereof.

10. Agent according to one of Claims 1 to 9, **characterized in that** it comprises direct dyes from the group of nitrophenylenediamines, nitroaminophenols, anthraquinones or indophenols, preferably in an amount of from 0.01 to 20% by weight, based on the total dyeing agent.

11. Agent according to one of Claims 1 to 10, **characterized in that** it comprises anionic, zwitterionic or nonionic surfactants.

12. Agent according to one of Claims 1 to 11, **characterized in that** it comprises oxidizing agents, in particular H₂O₂, in an amount of from 0.01 to 6% by weight, based on the application solution.

13. Agent according to one of Claims 1 to 12, **characterized in that** it comprises ammonium or metal salts chosen from the group of formates, carbonates, halides, sulphates, butyrates, valerates, caprbates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese; iron, cobalt, copper or zinc.

14. Use of a combination according to Claim 1 of dicarbonyl compounds with the formula I and CH-acidic compounds and/or compounds with a primary or secondary amino group or a hydroxy group, and/or physiologically compatible salts thereof as dyeing component in dyeing agents, in particular hair dyeing agents.

15. Method of dyeing keratinous fibres, in particular human hair, in which a dyeing agent comprising a combination according to Claim 1 of dicarbonyl compounds with the formula I and CH-acidic compounds and/or compounds with a primary or secondary amino group or a hydroxy group, and/or physiologically compatible salts thereof, and customary cosmetic ingredients, is applied to the keratinous fibres, is left on the fibres for a certain amount of time, usually about 30 minutes, and then rinsed out again or washed out with a shampoo.
